# EUROPEAN PATENT APPLICATION

(11) **EP 2 397 515 A1**
(43) Date of publication of application: **21.12.2011**
(21) Application number: 09839920.7
(22) Date of filing: 14.12.2009
(51) Int. Cl.: C08K 5/101, A61K 8/06, A61K 8/368, A61K 8/37, A61K 8/29

(54) **COSMETIC COMPOSITION FOR THE CARE AND CORRECTION OF TELANGIECTASIAS**

(30) Priority: 10.02.2009 ES 200900368
(71) Applicant: Laboratorios Starga, S.L., 30011 Murcia (ES)
(72) Inventor: SUÑÉ NEGRE, José Mª, E-30011 Murcia (ES); PICÓ GRAU, José Ramón, E-30011 Murcia (ES); ROIG CARRERAS, Manuel, E-30011 Murcia (ES); FUSTER GARCÍA, Roser, E-30011 Murcia (ES); IZQUIERDO GADEA, Mª Angeles, E-30011 Murcia (ES)
(74) Representative: Isern-Jara, Nuria
(86) International application number: PCT/ES2009/070582
(87) International publication number: WO 2010/092198

(57) **Abstract**

The present invention relates to a dermocosmetic composition of localised topical application in cream and/or emulsion form, to the procedure for obtainment thereof together with use thereof for care and correction of telangiectasias and to the method of administration thereof.

## Description

### OBJECT OF THE INVENTION

This invention generally relates to the field of cosmetics, more specifically to the field of topical care of telangiectasias or spider veins.

Specifically, this invention relates to a dermocosmetic composition for topical application in cream and/or emulsion form, the procedure for obtainment thereof together with use thereof for the treatment of telangiectasias.

### BACKGROUND TO THE INVENTION

Varicose veins appear when the venous valves that prevent the reflux of blood do not function correctly or when a vein wall weakens. As the valves do not function, the blood does not circulate well and the veins gradually expand, and in time they become visible and palpable, with consequences beyond those aesthetic, such as changes affecting the colour of the skin, pain, swelling of the limbs, etc.

It is not known exactly what causes the venous valves to weaken and deteriorate, but the factors that may contribute to their appearance are known. Obesity and weight fluctuations, ageing, leg injuries, pregnancy, a sedentary lifestyle and incorrect postures, constipation, alcohol and tobacco, hormones (oral contraceptives and hormone replacement therapies), hereditary factors, etc.

When these alterations only affect the skin capillaries they are called spider veins or telangiectasias. It is purely a cosmetic problem, and only in a small number of cases does it cause any discomfort in the form of a smarting sensation. When they appear, this generally associated with either hereditary factors or hormone and weight changes.

They are erroneously confused with varicose veins and they are often caused by the stimulating effect that the females hormones have upon the growth of new arterial blood vessels. Venal blockage is due to the direct effect of vasodilatation of the veins and to the increase in local blood flow in several vascular beds.

According to the Sociedad Española de Angiología y Cirugia Vascular (SEACV) (Spanish Angiology and Vascular Surgery Society), varicose veins affect 19.7 % of men and 39.8 % of women and it is expected that by 2010, 33 % of the population will be affected by varicose illnesses.

At present, there are different treatments available for relieving or curing these troubles. One of the treatments involves surgical methods such as stripping, which involves the removal of the entire varicose system. The endolaser, which removes by heat, veins larger than 2 or 3 millimetres. Ambulatory phlebectomy, which requires a local anaesthetic and involves making small incisions and extracting the varicose stretch. All these methods of ablative surgery can have side effects such as pain, blotches on the skin, burns, hiccough or hyper-pigmentation, in addition to the inconvenience that is caused by the patient having to undergo surgery.

That is why, in recent years, great importance has been attached to the non-surgical methods practiced, such as specialisation in aesthetic and dermatological medicine such as sclarotherapy, which consists of injecting a saline or chemical solution into the affected vein, which hardens it, preventing it from filling up with more blood, causing the blood flow to reach the heart via other veins. In this case, the veins that receive the injection dry in the course of time and disappear and the cicatricial tissue is absorbed by the body. In addition, this treatment requires 4 to 10 punctures to be made.

Another method is the vasculight laser, which is a transcutaneous laser that is recommended for veins no greater than 2 to 3 mm in diameter. Radiofrequency therapy is also being practiced. It involves closuring the vein by subjecting it to the heat transmitted by a small needle.

A system has recently been developed that enables sclerosis and radiofrequency to be applied simultaneously, thereby reducing the treatment time and giving favourable results. The IPL (Intense Pulsed Light): IPL is a revolutionary medical technology that by means of a special light makes it possible to remove the varicose veins and other small veins on the skin to be removed, red birth marks, and all kinds of dark pigments such as senile blotches, sun spots and freckles. The flashes of intense light applied to the skin do not cause any pain and remove the small veins on the face and on the body, and there is also the advantage of it not being necessary to use an anaesthetic or analgesic medication.

There are different patents registered for the aforementioned therapies, and these describe and protect each one of the existing methods in the state of the art. Thus, we can find the Spanish patent ES9800055, relating to a device for the application for anti-varicose treatment that is based upon injecting sclerosant products cooled to a very low temperature by carbonic liquid, and which is characterised by the fact that it is equipped with means for coupling and extracting liquid carbon from a tank that enable the liquid carbon to be applied at atmospheric pressure in a syringe containing the sclerosant product to be injected into the varicose vein without burning the user.

Similarly, the international patent W09400064ES, relates to an injectable microfoam that contains a sclerosant agent.

There is also the Spanish patent ES9701586, which relates to an endoluminal electro-coagulator for varicose veins surgery, integrated by an electrocoagulator capacity tip and sections that are essentially spherical, located on the distal end of a transmitting wire connected to a power supply. This electro scalpel or laser, is covered at the proximal end by an insulated sheath whose size and flexibility are suitable for percutaneous insertion. This device is designed to be used for operating on veins or vessels by electrocoagulation.

The Spanish patent ES200102185 is also comprised in the state of the art, and relates to a pocket laser device for the endoluminal treatment of varicose veins and other endocutaneous problems, composed of a small, rectangular body that has a liquid crystal display with digital sections to indicate start up; laser trip time with continuous or intermittent sequences; heat-related times and complete fluidity and, separately, a switch for start-up; on one of the long sides it has a calibration device and a Doppler volume output switch; on the other side it is equipped with a connector for video capillarity; a connection for a computer (PC) and a connection for an ecograph or ecodoppler and a scanner and at the bottom it has an outlet for a doppler gauge and an outlet for an optical fibre connection with a very low section for a major innovative adaptation to a hypodermic syringe, and a nozzle with blunt section and holes at the distal end.

Similarly, the patent P200303004 relates to the use of a prior injection of polydocanol in the form of foam for the painless removal of varicose veins by laser. This use consists of a combination of 1) prior treatment of the vascular injuries by injecting a hypermolar hyperalcoholic substance that can be injected into the vein; 2) injecting said substance in the form of a microfoam; and 3) applying to the veins the emission from an Nd-Yag laser in its basic emission (or applying any other laser that emits with a wavelength close to the resonant of the injected substance). In particular, this patent refers to said use when the substance used as the injectable substance is the polydocanol in the form of microfoam. The injection of said hydroalcoholic substance in the form of microfoam makes it possible to reduce the fluidity of the laser by 40%.

There are also other options such as preventive or ameliorative treatments of the physical discomfort caused by varicose veins, such as the use of reductive or compressive techniques that make it possible for the lower limbs to rest. In this sense, there is the Spanish utility model U0001032, which protects a rubber device for varicose veins, open and perforated, with a zip seal, with or without a separator. There is also the Spanish patent ES0164288, which protects a procedure for manufacturing in an elastic seamless rubber medium for varicose veins.

In addition to the treatments described above, there are also medical treatments such as administering phlebo-medications. However, these medicaments are not covered by the national insurance system and in view of the benefit-risk balance, most of the medications are used only for the treatment of diabetic retinopathy.

With the current state of the art, although there is topical therapy for this condition, it is very limited and only very old topical formulations can be found, such as, for example, Spanish patent ES9202020, which protects a gel that has been especially devised for varicose vein therapy by applying a light massage once a day that reduces inflammation. The invention also concerns the procedure for obtaining the anti-varicose gel. The main active components of the gel are a mixture of horse chestnut (glycolic extract of *Aesculus hippocastanum*), white hawthorn (glycolic extract of *Crataegus oxicantha*), Hamamelis (glycolic extract of *Hamamelis Virginia)*, carbopol (carboxyvinylic polymer), trietalomine, cremalys (perfume), dandelion (*Taraxacum officinalis*) and haragfito (*Harpagophytum procumbens*), and using in lesser proportions, as a preservative, katon CG (a mixture of two isothiazolinones: 5-chloro-2-methyl-4-isothiazolin 3-1 and 2methyl-4-isothiazolin 3-1), and all of this in a solution of isopropylic alcohol and water.

Also comprised in the state of the art is the Spanish patent ES9402228, relating to a rapid-action cream_against varicose veins, characterised by the fact that it is made on the basis of a mixture of Camphor, Lard and Bicarbonate, prepared en a *bain marie,* to which extra virgin olive oil from the first pressing and powdered sulphur is added, for a mixture in the same medium, until a consistent cream is obtained.

However, when we tried to find documents of the state of the art aimed specifically at telangiectasias or spider veins, we only found the Spanish patent ES9600773, although, like most of the documents mentioned above, it refers to an invasive treatment with the disadvantages that are inherent to these types of methods. This document of the state of the art specifically refers to a cryogenic syringe, of the type used to inject liquids at temperatures below 0 °C, for the removal of telangiectasias, capillaries, unpleasant varicose veins, which consists of a syringe and four concentric chambers, the internal one being the one that transmits the cold to the liquid to be injected, a chamber of carbonic snow that by means of suitable holes is linked to a valve chamber for releasing gas that, in turn, is surrounded by a chamber or insulated lining.

Therefore, there is a need to find a non-invasive, safe, effective, rapid and efficacious cosmetic care, for the cosmetic care of telangiectasias, which makes it easy for the patient to correctly and comfortably administer it, without it implying a health risk, since it is a corrective and repairing product for topical application on the skin.

Thus, this invention overcomes the drawbacks of the state of the art, being a dermocosmetic product for localised topical application on telangiectasias (non-invasive) that enables the patient to administer it in a controlled manner without the safety and toxicity problems that are inherent to chemical treatment and to treatments using drugs administered orally, which derive from their metabolisation and removal from the body.

Therefore, the object of this invention relates to a dermocosmetic preparation whose purpose is to care for and repair surface varicose veins that are unsightly and telangiectasias.

### DETAILED DESCRIPTION

At present, varicose veins and telangiectasias are very frequent problems, with the discomfort that affects the patients, with regard to their physical state, as well as the limitations that are caused, often restricting their activities, and preventing them from carrying out certain functions at work, sports, etc. In this sense, with an aim to relieving to the extent that this is possible such discomforts and the harmful consequences of varicose veins, especially because of their unsightliness, in accordance with the object of this invention a cream with dermocosmetic action for topical application is proposed, that is very advantageous for minimising the abovementioned effects as well as the unsightliness caused by the telangiectasias or spider veins.

This cream does not contain corticoids, which also means that it can be used continuously without any side effects such as atrophy of the skin and the appearance of stretch marks and cracks.

This invention relates to a dermocosmetic composition in cream form that contains an amount of cosmetically effective stearyl glycyrrhetinate, tocopheryl nicotinate, nicotinato de tocoferilo, talc, Karite butter, and titanium dioxide.

Likewise, this invention protects the method of application of said composition on the skin, comprising the stages of taking a sample of the dermocosmetic composition by using an application device, applying it in a localised manner on the spider vein (or telangiectasia) and applying heat onto the telangiectasia.

Likewise, this invention protects the procedure for obtaininment of said composition together with topical use thereof for the treatment of telangiectasias.

With respect to the method for obtaining the cream that is the object of this invention, the method is as follows:

### PHASE (A) AQUEOUS:

Weigh the distilled water in a beaker, add the maltitol with the aid of a magnetic agitator and shake with the magnetic agitator until it has completely dissolved. Add NIKKOSOME OS (squalene + glycerine + hydrogenated lecithin), and optionally hydrolyzed silk and shake until they are completely dissolved. Store covered until used.

### PHASE (B) ALCOHOLIC:

Weigh ethanol (96°) in a beaker, weigh propylenglycol, diethylenglycol monoethyl ether, add and shake in a magnetic agitator until dissolved. Weigh the rest of the components (preservatives and silicone) and add them one by one until they are completely dissolved. Store covered until used.

### PHASE (C) INSOLUBLE PHASE:

Weigh each one of the components (dye, talc and titanium dioxide) and transfer to a mortar. Mix them uniformly and pass the mixture through a sieve with a mesh width of 0.600 mm. Transfer the mixture to the mortar again, homogenise and store covered until used.

### PHASE (D): tocopheryl nicotinate

Weigh tocopheryl nicotinate in a watch glass. Keep it away from the light and air. Store until used.

### PHASE (O): OILY

Weigh petroleum jelly in a 250 ml beaker and melt it in a bath. Weigh one by one each of the rest of the components for this phase (liquid paraffin, cyclomethicone pentamer, stearyl glycyrrhetinate, polymethylsilsesquioxane, tribehenin, propylene glycol dicaprylate, cholesterol esters and lanoesterol, sorbitan laurate and Karite butter) and heat to 80°C (±2°C) and add this to the glass that contains the petroleum jelly. Heat up to 80°C (±2°C) until all the components have completely blended. Keep the mixture melted at 80°C (± 2°C).

This cream owes its beneficial properties to the selection and proportion of each one of its constituent components.

For example, the glyciretinic acid is a complex triterpene that is used locally for the treatment of non-infectious inflammatory skin discomforts, but it is also a powerful inhibitor of the enzyme 11-b-hydroxisteroid dehydrogenase that renders cortisol inactive. In fact, it has been demonstrated that the concomitant application of glyciretinic acid together with hydrocortisone enhances the latter's activity on the skin. Another property of glyciretinic acid is that it can esterify with stearylic alcohol, which is conducive to its absorption through the skin, the stearylic alcohol acting as a propellant, providing an emollient, dampening and tensioactive effect.

Stearyl glycyrrhetinate, a derivative of glycyrrhetinic acid is involved in the bleaching of the skin, so it is used in sun cream preparations because it has a melanogenesis inhibiting effect. In this sense, for the purpose of this invention, stearyl glycyrrhetinate has been selected as an active compound because it increases the solubility of lipids and has antiinflammatory properties at low concentrations.

Tocopheryl nicotinate is a substance with hypolipemiant and vasodilating properties, which is why it is frequently used to treat hyperlipidemia and peripheral vasculopathy (atherosclerotic or occlusive vasculopathy, as well as vein disorders secondary to atherosclerosis, vasospasm or thromboembolus) as well as cerebrovascular diseases.

For the object of this invention, the tocopheryl nicotinate favours oxygenation of the pilous bulb and cutaneous microcirculation, and also has an anti-oxidant and conditioning function of the skin, not being irritant nor sensitizing.

Purified talc powder, used externally, relieves irritation and prevents rubbing. It is generally mixed with starch and zinc oxide, to increase the absorption of moisture. Purified talc powder is also used as a lubricating and diluting excipient in the manufacture of tablets and capsules and to bleach liquids. The talc is also used as a sclerosing agent for malignant haemorrhages and in recurring spontaneous pneumothorax.

In the field of cosmetics, talc is used to facilitate application and dispersion, and also offers properties such as sliding and adhesion. Its covering power and its ability to absorb moisture are relatively low. Therefore, it is often combined with other products such as kaolin and zinc oxide.

Titanium dioxide is one of the whitest chemical substances that exist: it reflects practically all incident visible radiation and retains its colour permanently. It is one of the substances with the highest refraction index, even in powdered form or mixed with other substances. For this same reason, it is very opaque and serves to protect from the sunlight. Titanium dioxide pigments are also used in cosmetics as an absorber of ultraviolet radiation in cosmetic products.

Titanium dioxide also has the advantage of being inocuous, of being compatible with the mucous membranes and the skin and of having good dispersion in organic solutions. This is why titanium dioxide is commonly used as an ingredient in the field of cosmetics. Titanium dioxide also acts on the skin in a similar way to zinc dioxide, and is used together with titanium peroxide and titanium salicylate in the treatment of diaper erythema.

It has been demonstrated that titanium dioxide is not absorbed upon topical application, i.e., the analysis of titanium dioxide pigments only found their presence on the corneal stratum of the skin.

Karite butter serves to protect the skin from drying, and is a moisturiser for both the skin and the hair. It also protects against heat rash, prevents and treats the ageing of the skin, stimulates the cell metabolism and prevents wrinkles from appearing.

Karite butter also helps to heal injuries, and is suitable for the treatment of scaly skin, for dry and cracked hands, as well as a treatment for ulcers, eczema and stretch marks on the skin.

### PREPARATION OF THE CREAM

Melt phase (0) in a suitable container, and in the same bath, heat Phase (A) to 80°C (±2°C).

With Phases (O) and (A) at the same temperature, shake Phase (O) by hand and add slowly while continuing to shake Phase (A). Remove the mixture from the bath while still shaking it.

When the mixture reaches 50°C and while still shaking, add Phase (D) (tocopheryl nicotinate) until it is completely blended.

Then, while still shaking, add Phase (B), until it is completely blended. At this point a base of white fluid cream that is well emulsified must be obtained.

Transfer the base obtained into the mortar, containing the Phase (C) and work the mortar by hand until a cream is obtained with a uniform colour and texture. Pour the cream obtained into a container and cover securely.

### Exemplary embodiments

The following specific examples here provided serve to illustrate the nature of this invention. The examples included are merely illustrative and are not to be interpreted as limitative of the invention as claimed.

A flesh-coloured uniform cream is obtained. 24 hours after it has been prepared, the consistency of the cream has increased until a paste is obtained that when applied to the skin forms a consistent and well adhered and water-resistant layer (over which water flows when the skin is put under a tap), but this layer must be easy to remove by rubbing with water and shower gel.

The cream obtained is suitable for topical application, having good organoleptic, hiding, bioadhesive and water resistance qualities.

According to one important aspect this invention relates to a dermocosmetic composition for topical application for telangiectasias that comprises stearyl glycyrrhetinate, tocopheryl nicotinate, talc, titanium dioxide and Karite butter.

In another important aspect, of a preferred embodiment, the dermocosmetic composition for topical application for telangiectasias, comprises at least the following compounds in the following amounts by weight with respect to the total for the components in the composition:
- Stearyl glycyrrhetinate from 0.5 % to 15.00 %
- Tocopheryl nicotinate from 0.1 % to 5.00 %
- Talc from 1 % to 10.00 %
- Titanium dioxide from 1 % to 20.00 %
- Karite butter from 0.1 % to 5.00 %

According to another important aspect this invention relates to a procedure for preparing a dermocosmetic composition for topical application for telangiectasias that includes the following steps:
a) Prepare an aqueous phase from distilled water and maltitol, NIKKOSOME OS and hydrolyzed silk,
b) Prepare an alcoholic phase from ethanol, propylene glycol, monoethyl diethylene glycol ether, preservatives (parabens) and sorbic acid and silicon,
c) Melt an oily phase from a petroleum jelly, liquid paraffin, cyclomethicone pentamer, stearyl glycyrrhetinate, polymethylsilsesquioxane, tribehenin, propylene glycol dicaprylate, cholesterol esters and lanoesterol, sorbitan laurate and Karite butter and heat to 80°C (±2°C),
d) Heat the aqueous phase from Stage a) to 80°C (±2°C) and mix with the oily phase from step c),
e) Keep shaking the mixture obtained from step d) until it reaches a temperature of 50°C,
f) Add the tocopheryl nicotinate while shaking, until it reaches 45°C,
g) Add the alcoholic phase to the mixture from step f) while still shaking, until a white primary emulsion is obtained.
h) Add the rest of the insoluble compounds (dye, talc and titanium dioxide) after homogenisation and pass it through a sieve with a mesh width of 0.600 mm.

According to another important aspect this invention relates to the use of the composition for the preparation of a dermocosmetic composition for the care and correction of telangiectasias, which is for topical application in cream and/or emulsion form and that is additionally used as a make-up composition to conceal said telangiectasias.

According to another important aspect, this invention relates to a method of administering said composition on the skin by using an application device selected from the group of: a brush, a spatula, cotton, gauze swab, sponge, tube with applicator. In a preferred embodiment heat is applied to the affected zone.

The source of heat can be solar, a heater, a stove and any heating equipment, an electric blanket, a hot water bottle, warm clothing and any source of natural or artificial heat.

According to another important aspect, this invention relates to a method of administration of the above-mentioned composition for telangiectasias, which includes the steps of taking a sample of the dermocosmetic composition with the application device, the localised application thereof onto the spider vein or telangiectasia and the application of heat to said telangiectasia.

## Claims

1. A dermocosmetic composition for topical application of telangiectasias **characterised in that** it contains stearyl glycyrrhetinate , tocopheryl nicotinate, talc, titanium dioxide and Karite butter.

2. A dermocosmetic composition for topical application on telangiectasias of Claim 1 **characterised in that** it comprises at least the following compounds in the following amounts by weight with respect to the total for the components in the composition:
• Stearyl glycyrrhetinate: from 0.5% to 15.00%
• Tocopheryl nicotinate: from 0.1% to 5.00%
• Talc: from 1% to 10.00%
• Titanium dioxide: from 1% to 20.00%
• Karite butter: from 0.1% to 5.00%

3. A procedure for the preparation of a dermocosmetic composition for topical application on telangiectasias of Claim 1 **characterised in that** it comprises the following steps:
a) Prepare an aqueous phase from distilled water,
b) Prepare an alcoholic phase from ethanol,
c) Melt an oily phase from a petroleum jelly, stearyl glycyrrhetinate and Karite butter and heat to 80°C (±2°C),
d) Heat the aqueous phase from step a) to 80°C (±2°C) and mix with the oily phase from step c),
e) Keep shaking the mixture obtained from step d) until it reaches a temperature of 50°C,
f) Add the tocopheryl nicotinate while still shaking, until it reaches 45°C,
g) Add the alcoholic phase to the mixture from step f) while shaking, until a white primary emulsion is obtained.
h) Add the rest of the insoluble compounds (dye, talc and titanium dioxide) after homogenising and pass it through a sieve with a mesh width of 0.600 mm.

4. Use of the composition according to Claims 1 and 2 to prepare a dermocosmetic composition for care and correction of telangiectasias.

5. Use of the composition according to Claims 1 and 2 to prepare a dermocosmetic composition for topical application in cream and/or emulsion form.

6. Use of the composition according to Claims 1 and 2 to prepare a dermocosmetic composition for make-up and concealment of telangiectasias.

7. Use of the composition according to Claims 1 and 2 to prepare a dermocosmetic composition for topical application, using a device for application selected from the group of: a brush, a spatula, cotton, gauze swab, sponge, tube with applicator.

8. Method for administering the composition according to Claims 1 and 2 for telangiectasias **characterised in that** it includes the steps of taking a sample of the dermocosmetic composition with the application, applying it in a localised manner on the spider vein or telangiectasia and applying heat on said telangiectasia.

9. Method for administering the composition according to Claim 8 **characterised in that** the source of heat is selected from the group formed by a natural source of heat or an artificial source of heat.

10. Method for administering the composition according to Claim 9 **characterised in that** the source of heat is selected from the group formed by solar heat, a heater, a stove, an electric blanket, a hot water bottle or warm clothing.
